Europäisches Patentamt

European Patent Office

Office européen des brevets

(11) Publication number: **0 188 628**
**A1**

# EUROPEAN PATENT APPLICATION

published in accordance with Art. 158(3) EPC

(21) Application number: 85903697.2

(22) Date of filing: 19.07.85

Data of the international application taken as a basis:

(86) International application number:
PCT/JP 85/00408

(87) International publication number:
WO 86/00932 (13.02.86 86/4)

(51) Int. Cl.⁴: **C 12 P 7/64**
// C12N9/20 ,(C12P7/64,
C12R1:44)

(30) Priority: 20.07.84 JP 149584/84

(43) Date of publication of application: 30.07.86
Bulletin 86/31

(84) Designated Contracting States: **AT BE CH DE FR GB IT
LI LU NL SE**

(71) Applicant: Suntory Limited, 1-40 Dojimahama 2-chome
Kita-ku, Osaka-shi Osaka-fu 530 (JP)

(72) Inventor: AMACHI, Teruo Oyobiseibutsukenkyusho,
Suntory Limited 1-1-1, Wakayamadai, Shimamoto-cho,
Mishima-gun Osaka 618 (JP)
Inventor: TANAKA, Takaharu Oyobiseibutsukenkyusho,
Suntory Limited 1-1-1, Wakayamadai, Shimamoto-cho,
Mishima-gun Osaka 618 (JP)
Inventor: OGURA, Kyoichi Oyobiseibutsukenkyusho,
Suntory Limited 1-1-1, Wakayamadai, Shimamoto-cho,
Mishima-gun Osaka 618 (JP)
Inventor: ASAMI, Sumio Oyobiseibutsukenkyusho,
Suntory Limited 1-1-1, Wakayamadai, Shimamoto-cho,
Mishima-gun Osaka 618 (JP)
Inventor: ISHIGOOKA, Hiroshi Oyobiseibutsukenkyusho,
Suntory Limited 1-1-1, Wakayamadai, Shimamoto-cho,
Mishima-gun Osaka 618 (JP)
Inventor: NAKAO, Masahiro Oyobiseibutsukenkyusho,
Suntory Limited 1-1-1, Wakayamadai, Shimamoto-cho,
Mishima-gun Osaka 618 (JP)

(72) Inventor: YOSHIZUMI, Hajime Oyobiseibutsukenkyusho,
Suntory Limited 1-1-1, Wakayamadai, Shimamoto-cho,
Mishima-gun Osaka 618 (JP)

(74) Representative: Ford, Michael Frederick et al,
MEWBURN ELLIS & CO. 2/3 Cursitor Street, London
EC4A 1BQ (GB)

(54) **PROCESS FOR PRODUCING FATTY ACIDS BY FERMENTATION.**

(57) A process for producing fatty acids used for wide applications in chemical inductries, such as industrial chemicals, medicines, perfumes, cosmetics, etc., which process comprises culturing bacteria belonging to the genus *Staphylococcus* and being capable of producing lipase in a medium containing fat or oil, or initiating the culturing of the bacteria in a medium not containing fat nor oil and adding fat or oil in the course of the culturing, to thereby produce fatty acids in a culture liquor, and collecting the fatty acids from the culture liquor. This process enables to produce highly pure fatty acids economically on an industrial scale.

- 1 -

## DESCRIPTION

TITLE OF THE INVENTION

Process for Preparing Fatty Acids by Fermentation

TECHNICAL FIELD

This invention relates to a process for preparing fatty acids used extensively in the chemical industry for the manufacture of, for example, industrial products, pharmaceuticals, perfumes, and cosmetics.

BACKGROUND ART

Processes involving a nonenzymatic decomposition of natural fats or oils, such as a process comprising synthesizing higher fatty acids via the oxidization of paraffins, a process comprising decomposing refined natural fats or oils under high pressure, the Twitchell decomposition process, and those involving enzymatic hydrolysis of natural fats or oils with lipase products, are known as processes for preparing fatty acids for use as industrial raw material.

In the synthesis process via the oxidization of paraffins, however, problems arise in connection with the product quality. In the process for preparing fatty acids involving nonenzymatic decomposition of natural fats or oils, the fatty acids once formed and impurities contained in the fats or oils used as the raw material, either decompose or react with each other to form various impurities resulting in the produced fatty acids becoming toxic, irritative, colored, or odorous so that they cannot be safely used for the manufacture of pharmaceuticals, cosmetics, and the like. On the other hand, the process for preparing fatty acids comprising hydrolyzing fats or oils with lipase may be considered an advantageous process for the preparation of high-purity fatty acids, in that the reaction takes place under mild conditions, and only fats and oils are specifically hydrolyzed without giving rise to harmful impurities during reaction.

- 2 -                    0188628

The lipase hydrolysis method, however, has an economic disadvantage in that lipase products are relatively expensive, and furthermore, the use of conventional lipase does not permit the fats or oils to be completely hydrolyzed resulting in a low yield of the fatty acids due to the presence of monoglyceride and diglyceride residues.  As a means of resolving this economic disadvantage, a method has been proposed to reduce the amount of lipase used by the addition to the hydrolysis-reaction system of a water-insoluble organic solvent during hydrolysis of fats or oils with lipase (Japanese Unexamined Patent Publication 59-91889), but this method is not necessarily considered practical.

DISCLOSURE OF THE INVENTION

The present invention provides a process for preparing fatty acids by the lipase hydrolysis method, which are free from the shortcomings of similar methods based on the prior art, and is particularly directed to using an enzyme capable of substantially completely hydrolyzing triglyceride to fatty acids and glycerine without allowing monoglyceride and diglyceride to remain, and to using the enzyme in the most economical manner.

This problem may be resolved by employing a process for preparing fatty acids characterized by culturing a lipase producing bacterium belonging to the genus Staphylococcus in a medium containing fat or oil, or by adding fat or oil during the culturing of that bacterium in a medium, and obtaining fatty acids from the culture medium.

In accordance with the present invention, it is possible to prepare fatty acids more economically than by the conventional method because of the substantially complete hydrolysis of fat or oil resulting in the absence of monoglyceride and diglyceride residues.

BRIEF DESCRIPTION OF THE DRAWINGS

Figure 1 is a gas-chromatogram of the fatty acids,

prepared in accordance with a process of the present invention (Example 1) wherein 1, 2, and 3 respectively represent the peaks of oleic acid, palmitic acid, and linolic acid;

Fig. 2 is also a gas-chromatogram of the fatty acids prepared in accordance with a process of the present invention (Example 2) wherein 1, 2, and 3 represent the same matter as in Fig. 1;

Fig. 3 and Fig. 4 are thin-layer chromatograms each showing various hydrolysates obtained by the hydrolysis of olive oil carried out in accordance with a process of the present invention using various strains of Staphylococcus capitis;

Fig. 5 shows thin-layer chromatograms of various hydrolysates obtained by the hydrolysis of olive oil carried out in accordance with a process of the present invention using various strains of Staphylococcus epidermidis;

Fig. 6 shows thin-layer chromatograms of the hydrolyzates obtained by the hydrolysis of olive oil carried out in accordance with a process of the present invention using various strains of Staphylococcus aureus;

Fig. 7 and Fig. 8 are thin-layer chromatograms showing the products obtained by the hydrolysis of triolein carried out using lipases derived from various strains of Staphylococcus capitis; and

Fig. 9 shows thin-layer chromatograms of the products obtained by the hydrolysis of triolein carried out using various commercially available lipases.

BEST MODE OF OPERATION FOR THE INVENTION

Microorganisms

Bacteria capable of producing lipase and belonging to the genus Staphylococcus are used in the processes of this invention. For example, Staphylococcus capitis, Staphylococcus epidermidis, and Staphylococcus aureus may be used. Where lipase derived from the bacteria

- 4 -                    0188628

belonging to the genus _Pseudomonus_, lipase derived from fungi belonging to the genus _Rhizopus_, and lipase derived from yeasts belonging to the genus _Candida_ are used to hydrolyze triglyceride, substantial amounts of monoglyceride and diglyceride are formed as the hydrolytic products resulting in a decline in the yield of fatty acids. On the contrary, where lipases deriving from bacteria belonging to the genus _Staphylococcus_ are used to hydrolyze triglyceride very little diglyceride is formed, and any amount of monoglyceride formed is extremely small. Particularly, where lipase deriving from _Staphylococcus_ _capitis_ is used to hydrolyze triglyceride, virtually no monoglyceride and diglyceride are formed. Accordingly, _Staphylococcus_ _capitis_ is most preferable as the microorganism for use in the process of this invention.

As examples of the strains of _Staphylococcus_ _aureus_ which may be used in the process of this invention, strains IAM 1011, IAM 1098, and IAM 12082 may be cited. As the strains of _Staphylococcus_ _capitis_, strains ATCC 27840, ATCC 27841, ATCC 27842, ATCC 27843, and T-1-1 (SAM 0001) may be cited. Of these microorganisms, those having the designation "IAM" are deposited at the Institute of Applied Microbiology, University of Tokyo; 1-1 Yayoi 1-chome, Bunkyo-ku, Tokyo, Japan, and are readily available, and those with the "ATCC" designation are deposited at the American Type Culture Collection at 12301 Parklawn Drive, Rockville, Maryland 20852, U.S.A., and also are readily available.

The _Staphylococcus_ capitis T-1-1 (SAM 0001) is a new strain isolated from the human scalp by the inventors, and possesses the following characteristics:

(A) Morphorlogy

(1) Size: Cocci ranging in diameter from 0.8 μm to 1.2 μm, and from 2 to 4 cocci found in clusters.

(2) Motility:          None.

(3) Sporulation:       Not observed.

(4) Gram Stain:        Positive.

(5) Acid-resistance:   None.

(B) Growth Characteristics

(1) Bouillon Agar Plate Culture:
    Colonies slightly convex, round, smooth
    surface, opaque white and small in size with
    diameters ranging from 1 mm to 3 mm.

(2) Bouillon Liquid Culture:
    Good growth; broth entirely turbid.

(3) Bouillon Gelatin Stab Culture:
    Gelatin not liquefied; growth linear.

(4) Bouillon Agar Slant Culture:
    Slightly convex, smooth surface, opaque
    white, and good growth.

(5) Litmus Milk:          No change in color, and no solidification or liquefaction observed.

(6) Methylred (MR) Test:          Positive.

(7) Voges-Proskauer (VP) Test:    Positive.

(8) Indole formation:             None.

(9) Hydrogen Sulfide Formation:   None.

(10) Amylolyzability:             None.

(11) Citric Aid Utilization:      Positive in Khozer-Christensen reaction.

(12) Inorganic Nitrogen Source Utilization:
    Nitrate utilized, but ammonium salts not
    utilized.

(13) Pigment-reducing Ability:    None.

(14) Urease Reaction:             Negative.

(15) Oxidase Reaction:            Negative.

(16) Catalase Reaction:           Positive.

(17) Growth Range:

pH 5 ∿ 9; temperatures 20°C ∿ 40°C;
optimum growing temperatures within 32°C
to 35°C range.

(18) Oxygen Requirement:          Facultatively
                                  anaerobic.

(19) Sugar Utilization:

Acid Formation (+):    D-glucose,
                       D-fructose,
                       D-mannose,
                       sucrose, and
                       glycerine.

Acid/Gas Formation (-):  D-galactose,
                         D-xylose,
                         L-arabinose,
                         maltose,
                         lactose, trehalose,
                         D-sorbitol,
                         inositol, and
                         starch.

Lactic acid is formed from D-glucose.

(20) Salt Resistance:    Salt-resistant (capable of
                         growth in 10% to 20%
                         saline solution)

(21) Lipase Activity:    Present.

(22) Lecithinase Activity:    Present.

(23) Coagulase Activity:    Absent.

(24) Phosphatase Activity:    Absent.

(25) Deoxyribonuclease Activity:    Present.

This microorganism, Staphylococcus capitis T-1-1
(SAM 0001) has been deposited with the Fermentation
Research Institute Agency of Industrial Science and
Technology, Ministry of International Trade and Industry,
at 1-1-3 Yatabe-cho Higashi, Tsukuba-gun, Ibaraki-ken,
Japan as FERM P-7723. Note, this microorganism was
transfered to international deposition under the Budapest
Treaty on July 11, 1985 as FERM BP-834.

## Culturing and Reaction

In accordance with the present invention, fats or oils are hydrolyzed by lipase produced by bacteria to form fatty acids. As examples of the mode of operation for the hydrolysis process, the following methods may be employed:

(1) Fat or oil suitable for use as the raw material is first introduced into a medium in which the bacteria can grow and the medium is inoculated with the bacteria. Then the hydrolysis of the fat or oil is conducted in parallel with the growth of the bacteria.

(2) The bacteria are first grown in a medium in which they can grow, followed by the addition to the medium of fat or oil which is the raw material. Then the hydrolysis of the fat or oil is conducted in parallel with the culturing.

In the process outlined in Paragraph (1) above, it is possible to initially add the entire amounts of the fat or oil to the medium, but it is also possible to commence the culturing after initially adding a portion of the fat or oil followed by the continuous or stepwise addition of the remaining portions of the component while monitoring the subsequent bacteria growth and the extent of hydrolysis of the added fat or oil.

In the process outlined in Paragraph (2) above, as in the process outline in Paragraph (1), the fat or oil may be added not only in a single dose, but also stepwise or continuously in the process outlined in Paragraph (2). In these instances, the time for the addition of the fat or oil (or for commencing the addition) may be either the initial phase of the culturing where the bacteria have begun to grow, or the phase where the growth of the bacteria is almost complete, or any arbitrary phase in between.

In the process of the present invention, any medium in which the bacteria can grow and produce lipase may be used. As a carbon source for the medium, glucose, fructose, sucrose, glycerol or the like may be used either singly or in combination, and as a nitrogen source, peptone derived from soybean casein or the like, yeast extract, or others may be also used either singly or in combination. Inorganic salts such as phosphate, magnesium salt, manganese salt, and common salt, or such growth-promoting compounds as vitamins also may be added.

For culturing, an inoculum prepared by preincubating the bacteria of the present invention in a liquid medium without fats or oils is used to inoculate a main culture medium with or without fats or oils for main culturing.

The culturing and reaction conditions vary with the strains used, but the temperatures should be in the range of 20°C to 40°C, preferably 30°C to 37°C, with the pH of the medium adjusted to 5.0 to 9.0, preferably 6.0 to 7.0. ·In any case, it is preferred that culturing be conducted using a liquid medium and under an aerobic condition arising from aeration, agitation, or shaking.

A term of culturing in the medium to which fat or oil is initially added is from about 20 hours to 70 hours, and where the fat or oil is added during culturing, the culturing is continued until the fat or oil is thoroughly hydrolyzed after the addition thereof is completed, or the reaction is continued under the conditions similar to those for the culturing.

Any types of fats or oils containing glycerides are suitable for use in the process of this invention. Vegetable fats and oils such as olive oil, coconut oil or castor oil, and animal fats and oils such as tallow, whale oil, or mixture thereof may be used. An amount of the fat or oil to be added varies with the activities of lipase formed in the medium and the other conditions, with preferable levels ranging from about 0.5 (v/v)% to about 5.0 (v/v)%.

Recovery of Fatty Acids

In accordance with the present invention, fatty acids accumulate in the cultured medium. The fatty acids may be readily refined by conventional methods such as extraction, fractional crystallization, column chromatography or the like, from the supernatant liquid obtained by filtration or centrifugation of the cultured broth to remove the bacterial cells.

Extraction of free fatty acids from the supernatant liquid is carried out with a low-polarity, water-immiscible organic solvent such as ethyl acetate, chloroform, or benzene. After the extraction, the solvent is then distilled-off under a reduced pressure and the residue is mixed with alcohol such as methanol or the like with stirring. The fatty acids then pass into the alcohol layer, leaving the fat or oil remaining in the residue. Purification of the fatty acids from the resulting alcohol-soluble fraction free from unhydrolyzed fat or oil may be carried out by such methods as pressing, fractional crystallization, or liquid-liquid extraction. Such purification also may be carried out by either normal phase column chromatography using silica gel or the like, or by reverse phase column chromatography using RP-18 or the like.

The following examples are provided solely for further illustration:

Example 1. Preparation of Fatty Acid By Staphylococcus Capitis; Variation Involving The Initial Addition of Oil

Two hundred ml. portions of a P medium (Yeast extract 0.5% (w/v); Polypeptone, (peptone manufactured by Takeda Pharmaceutical Industry), 1% (w/v); Glucose 0.1% (w/v); NaCl 0.5% (w/v), and pH adjusted to 6.3) were added to 500-ml flasks which were then sterilized at 120°C for 15 minutes. Three flasks thus prepared were inoculated with Staphylococcus capitis T-1-1 and the flasks were shaken at 240 rpm for 24 hours at 30°C on a rotary shaker to produce inocula.

Thirty l. of a medium containing 5% (w/v) of AFF peptone (a soybean protein hydrolyzate manufactured by Ajinomoto & Co.), and 0.5% (w/v) of NaCl and adjusted to pH 6.3 were added to 50 ℓ jar fermentors which were then sterilized at 120°C for 15 minutes. The medium was inoculated with the inocula followed by the addition of 300 ml of olive oil previously sterilized at 120°C for 15 minutes. Culturing was carried out at 30°C for 30 hours with agitation at 300 rpm and aeration at 1.0 vvm.

The cultured broth obtained was centrifuged to remove the bacterial cells. Thirty liters of ethyl acetate was added to the supernatant liquid and the mixture was stirred for 1 hour. After stirring, the mixture was allowed to stand for 1 hour to achieve a phase separation. The ethyl acetate layer was removed, concentrated, and evaporated to dryness under a reduced pressure. Three liters of methanol were added to the residue and the mixture was stirred overnight at 5°C. This mixture was centrifuged to obtain a methanol layer. The methanol layer was then concentrated to dryness under a reduced pressure and the residue was dissolved in 2 liters of n-hexane. The n-hexane solution was then concentrated to dryness under a reduced pressure. The residue was dissolved in 100 milliliters of benzene and placed on the silica gel column (5 x 55 cm), washed with 1 liter of benzene, and eluted with 2 liters of a benzene-chloroform mixture (1:1). The column was then eluted with 3 liters of chloroform and 100 g of a fatty acid mixture obtained therefrom.

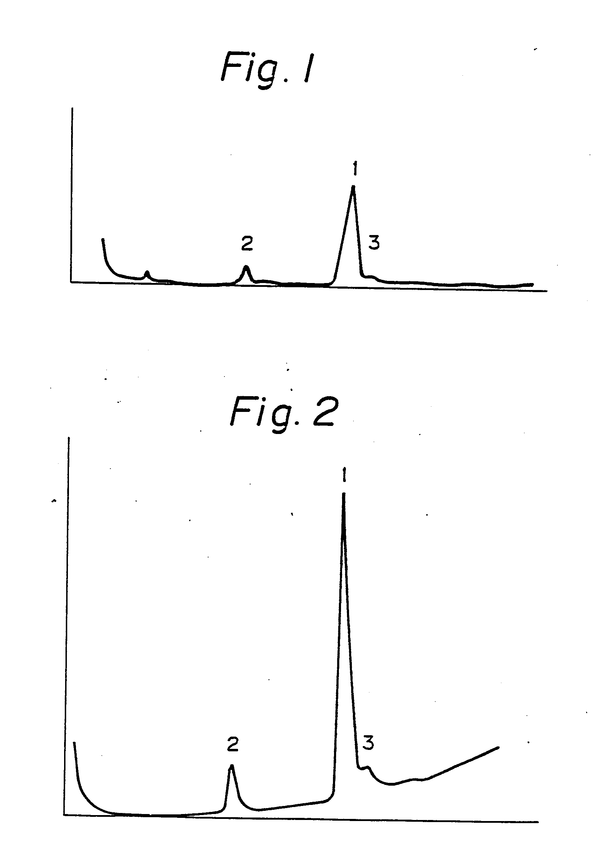

A gas-chromatogram of the fatty acid mixture obtained by the method described above is shown in Fig. 1.

Seventy-six grams of oleic acid were obtained by resubjecting the product of the invention obtained by the method described above to silica gel gas chromatography and collecting the oleic acid fractions.

Example 2.    Preparation of Fatty Acids
Using Staphylococcus Capitis; Variation
Involving The Addition of Oil After Culturing

As in Example 1, <u>Staphylococcus</u> <u>capitis</u> (ATTC-27840) was cultured in 200 ml of a P medium with shaking on a rotary shaker. A 14-liter jar fermenter was charged with 7 liters of a main culture medium prepared as described in Example 1 and sterilized under pressure for 15 minutes at 120°C in advance. The main culture medium was inoculated with the first cultured medium and cultured for 24 hours at 30°C with agitation at 300 rpm and aeration at 2.0 vvm. One-hundred-milliliters of olive oil was added to the cultured broth thus obtained and the whole broth was agitated for 12 hours at 30°C and at 300 rpm. The product obtained was purified by the method described in Example 1 and 38 g of a free fatty acid mixture was obtained.

A gas-chromatogram of the product of the invention obtained by this method is shown in Fig. 2.

<u>Example 3.</u>    <u>Hydrolysis of Olive Oil Using</u>
<u>Various Strains of The Genus Staphylococcus</u>

One percent olive oil was added to a 10% AFF peptone medium (10% AFF peptone and 0.5% NaCl). The medium was inoculated with the following strains and cultured for 3 days with shaking:

(1)  <u>Staphylococcus</u> <u>capitis</u> ATCC 27840
(2)  <u>Staphylococcus</u> <u>capitis</u> ATCC 27841
(3)  <u>Staphylococcus</u> <u>capitis</u> ATCC 27842
(4)  <u>Staphylococcus</u> <u>capitis</u> ATCC 27843
(5)  <u>Staphylococcus</u> <u>capitis</u> T-1-1 (FERM
                                        P-7723)
(6)  <u>Staphylococcus</u> <u>aureus</u>  IAM 1011
(7)  <u>Staphylococcus</u> <u>aureus</u>  IAM 1098
(8)  <u>Staphylococcus</u> <u>aureus</u>  IAM 12082
(9)  <u>Staphylococcus</u> <u>epidermidis</u> No. 2
(10) <u>Staphylococcus</u> <u>epidermidis</u> No. 3
(11) <u>Staphylococcus</u> <u>epidermides</u> No. 5
(12) <u>Staphylococcus</u> <u>epidermidis</u> No. 8
(13) <u>Staphylococcus</u> <u>epidermidis</u> No. 11

Samples were taken on days 2 and 3 during the incubation and fatty acids were extracted with
chloroform. The composition of the fatty acids was
investigated by thin-layer chromatography. The system
used for the thin-layer chromatography was as follows:

Solvent System: Chloroform-Acetone (96:4)

Color Development: 1% $Ce(SO_4)_2$ -10% $H_2SO_4$ heating

Thin-layer Plate: Silica gel, Kiesel gel 60 $F_{254}$ (manufactured by Merck & Co.)

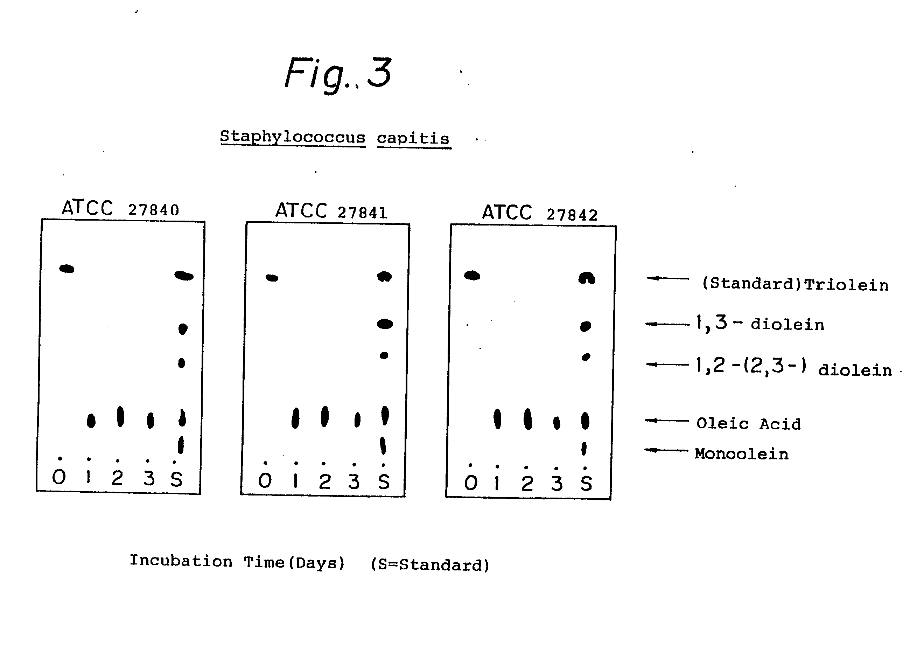

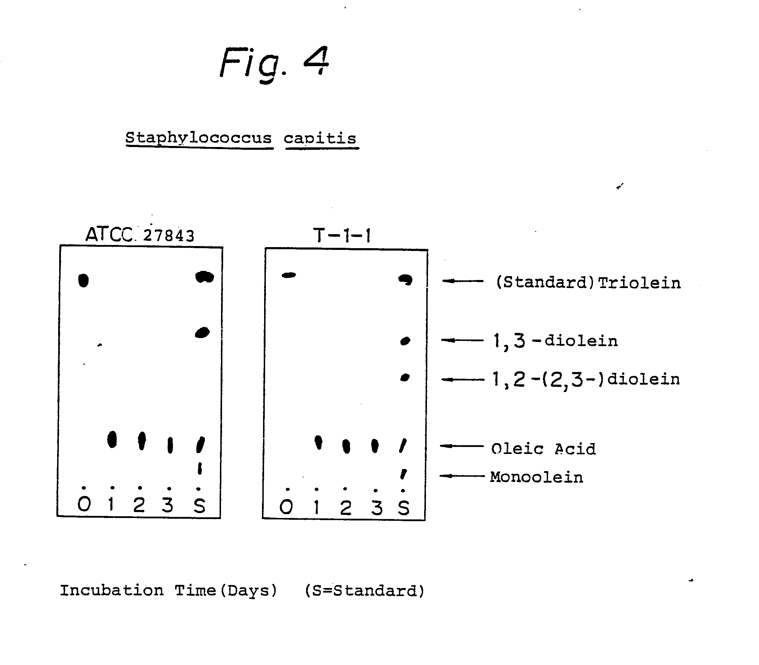

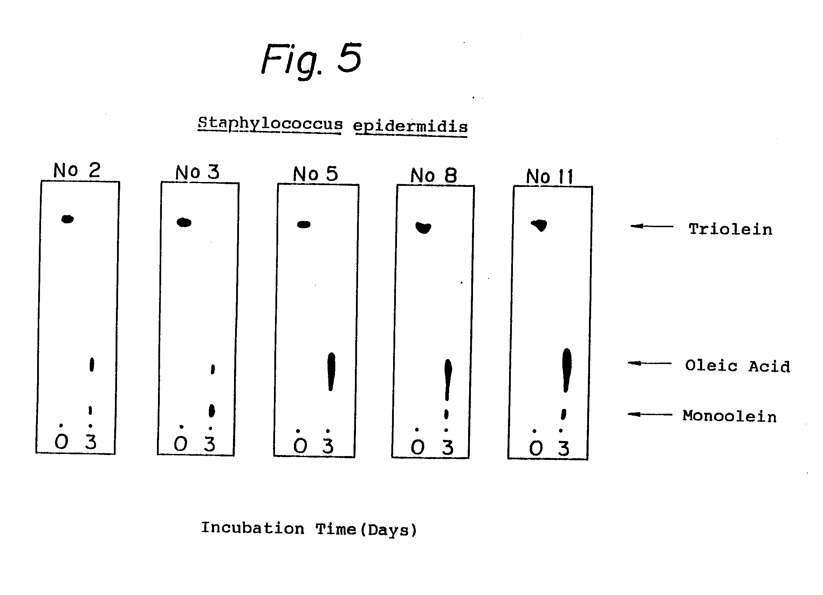

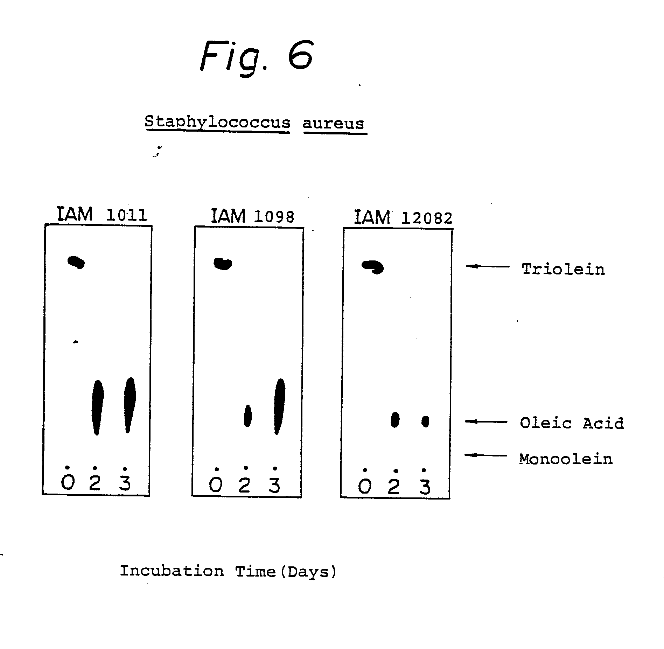

The results are shown in Figs. 3, 4, 5, and 6.
As can be seen from these figures, diglyceride (1,3
diolein and 1,2-(2,3-)diolein) was not detected and very
little monoglyceride (monoolein) was detected with
fatty acid (oleic acid) being predominantly formed.

Example 4. Hydrolysis of Triolein by Lipase
Derived from Strains of The Genus
Staphylococcus and by Lipases Derived from
Other Microorganisms

Bacteria of the genus Staphylococcus described
in Example 3 were cultured on the same manner as that
described in Example 3 (except that olive oil was not
added to the medium). After culturing, the cultured
broth was centrifuged to remove the bacterial cells and
the supernatant solution obtained was used as enzyme
solutions. The lipase activities of these enzyme
solutions were as follows:

| No. | Strain | Lipase Activity (U/ml) |
|---|---|---|
| | Staphylococcus capitis | |
| 1 | ATCC 27840 | 11.7 |
| 2 | ATCC 27841 | 13.5 |
| 3 | ATCC 27842 | 4.6 |
| 4 | ATCC 27843 | 12.4 |
| 5 | T-1-1 | 6.1 |
| | Staphylococcus aureus | |
| 6 | IAM 1011 | 3.2 |
| 7 | IAM 1098 | 1.0 |
| 8 | IAM 12082 | 2.1 |
| | Staphylococcus epidermidis | |
| 9 | No. 2 | 21.0 |
| 10 | No. 3 | 9.4 |
| 11 | No. 5 | 21.9 |
| 12 | No. 8 | 10.1 |
| 13 | No. 11 | 7.4 |

The above lipase activities were determined in the following manner:

One milliliter of olive oil was added to test tubes (φ 23 x 200 mm) each containing 5 ml of a reaction mixture comprising 25 mM Tris-HCl buffer (pH 8.0)/10 mM $CaCl_2$. The mixture was vigorously stirred with a mixer, after which 0.5 ml of the enzyme solution was added. The reaction was carried out for 30 minutes at 30°C, during which period the test tubes were shaken on a reciprocating shaker at 300 rpm. The reaction was then stopped by the addition of 20 ml of ethanol and the fatty acids formed titrated with 0.05 N aqueous sodium hydroxide solution. The control values were likewise determined by titrating the solution obtained by first adding ethanol to a reaction mixture of the composition and then adding an enzyme solution to the same. According to a proposal by Enzyme Nomenclaure; recommendations (1978) of the nomenclature committee of the International Union of Biochemistry (I.U.B.), Academic Press, a unit of lipase activity is defined as the amount of lipase required to liberate 1 μ mole of fatty acid per minute under the reaction condition.

Next, each of the enzyme solutions, except for those whose lipase activities were below 3 units, was diluted to 3 U/ml of enzyme activity.

On the other hand, another set of enzyme solutions were prepared for comparison purposes by dissolving commercially available enzyme preparations (Boehringer Mannheim Co.) to 3.0 U/ml. The following three different enzyme preparations were used:

     (1)   Lipase derived from <u>Rhizopus</u> <u>arrhizus</u>

     (2)   Lipase derived from <u>Candida</u> <u>cylindracea</u>

     (3)   Lipase derived from <u>Pseudomonas</u> sp.

When preparing the enzyme solutions, 0.1 M MOPS-HCl buffer (pH 6.0) was used for the enzyme preparation (1) and (2), and 25 mM Tris-HCl buffer (pH 8.0, 10 mM $CaCl_2$) was used for the enzyme preparation (3).

Ten microliters of triolein was added to 1 ml of the enzyme solution obtained from the cultured broths of the present invention and containing 3.0 U/ml of lipase and the solutions were shaken at 30°C. Samples were then taken at 0, 10, 30, 60, and 120 minutes and subjected to extraction with chloroform. Two microliters of the chloroform layer was assayed by thin-layer chromatography as described in Example 3. The same procedure as above was used for the commercially available enzyme preparations except that 1 ml of a

0188628

- 15 -

3.0 U/ml enzyme solution was used.

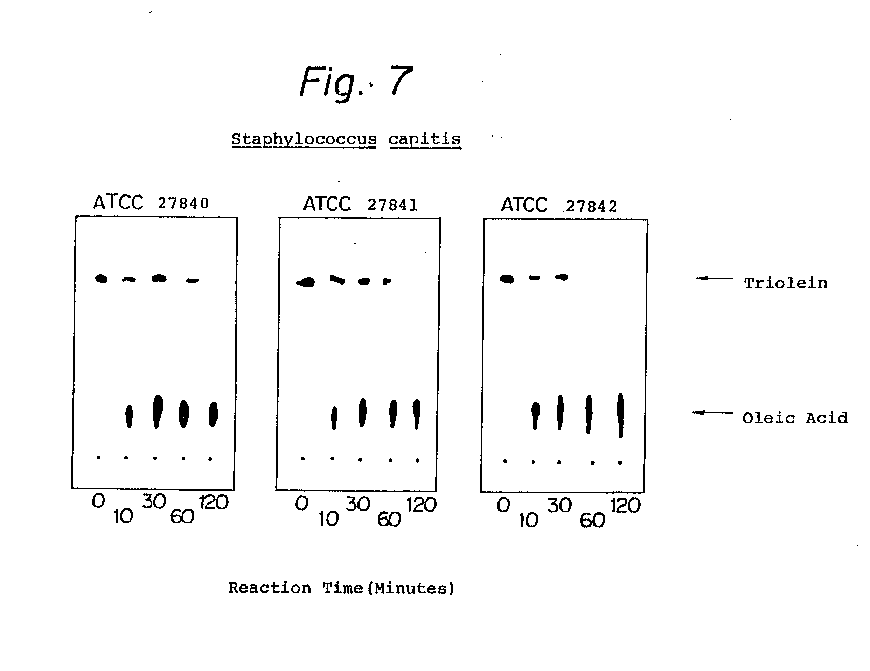

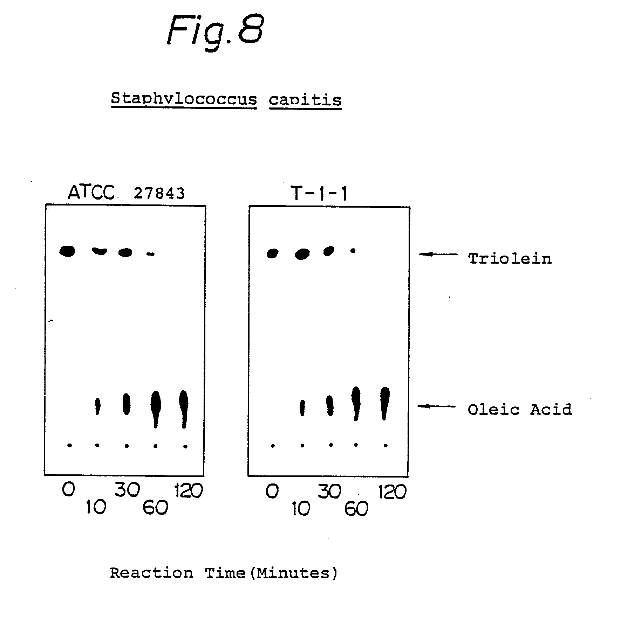

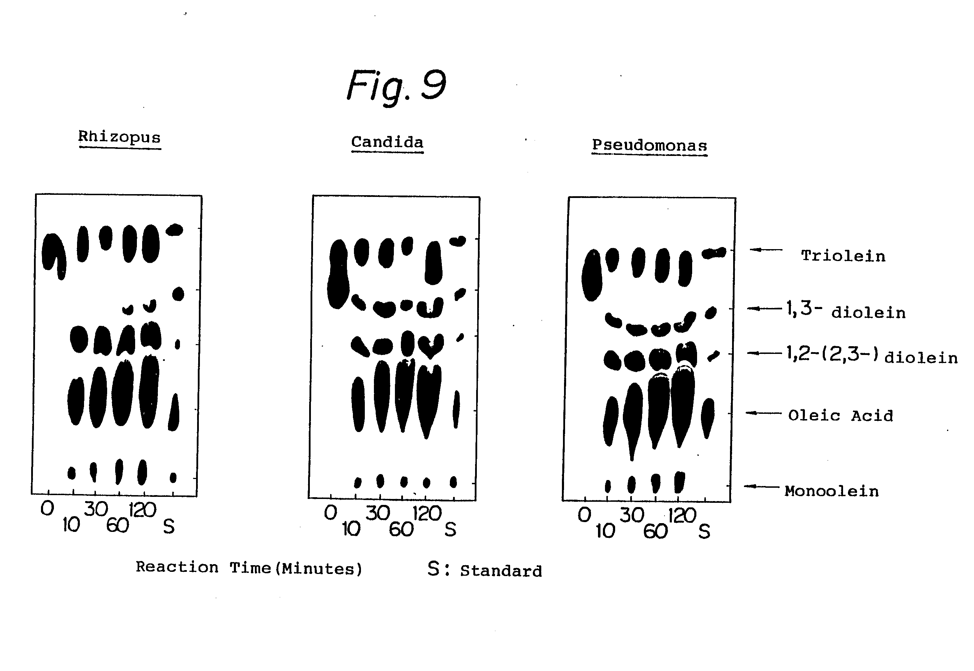

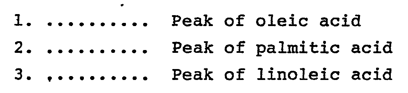

The results obtained are shown in Figs. 7, 8, and 9. As can be seen from these figures, where triolein was hydrolyzed by the commercially available enzyme, substantial amounts of monoolein and diolein were formed, but where Staphylococcus-deriving enzymes of the present invention were used, very little of such substances were formed. Particularly, when the enzyme derived from Staphylococcus capitis was used, monoolein and diolein were not detected at all.

CAPABILITY FOR EXPLOITATION IN INDUSTRY

This invention is useful for economically preparing fatty acids having a high purity on an industrial scale.

- 16 -

## CLAIMS

1. A process for preparing fatty acids characterized by the steps of: incubating lipase-producing bacterium of genus <u>Staphylococcus</u> in a medium containing fat or oil, or first commencing culturing said bacterium in a medium containing no fat and oil and then adding fat or oil during culturing, to form fatty acids in the cultured broth, and recovering the fatty acids from the broth.

2. A process according to Claim 1 wherein the bacterium of genus <u>Staphylococcus</u> used is <u>Staphylococcus</u> <u>capitis</u>.

3. A process according to Claim 2 wherein the strain of <u>Staphylococcus</u> capitis used is <u>Staphylococcus</u> <u>capitis</u> T-1-1 (SAM 0001) (FERM P-7723).

*Fig. 1*

*Fig. 2*

# Fig. 3

Staphylococcus capitis

ATCC 27840    ATCC 27841    ATCC 27842

← (Standard)Triolein

← 1,3 – diolein

← 1,2 –(2,3–) diolein

← Oleic Acid

← Monoolein

O 1 2 3 S    O 1 2 3 S    O 1 2 3 S

Incubation Time (Days)    (S=Standard)

*Fig. 4*

Staphylococcus capitis

ATCC 27843          T-1-1

—— (Standard) Triolein

—— 1,3-diolein

—— 1,2-(2,3-)diolein

—— Oleic Acid

—— Monoolein

0  1  2  3  S      0  1  2  3  S

Incubation Time (Days)    (S=Standard)

Fig. 5

Staphylococcus epidermidis

Triolein

Oleic Acid

Monoolein

No 11

No 8

No 5

No 3

No 2

Incubation Time (Days)

# Fig. 6

Staphylococcus aureus

Incubation Time (Days)

Fig. 7

Staphylococcus capitis

ATCC 27840          ATCC 27841          ATCC 27842

← Triolein

← Oleic Acid

Reaction Time (Minutes)

6/9

0188628

# Fig.8

Staphylococcus capitis

| ATCC 27843 | T-1-1 |
|---|---|

← Triolein

← Oleic Acid

ATCC 27843: 0 10 30 60 120

T-1-1: 0 10 30 60 120

Reaction Time (Minutes)

# Fig. 9

Rhizopus

Candida

Pseudomonas

← Triolein

← 1,3- diolein

← 1,2-(2,3-) diolein

← Oleic Acid

← Monoolein

Reaction Time (Minutes)     S: Standard

List of Reference Numbers

1. ........... Peak of oleic acid
2. .......... Peak of palmitic acid
3. ,......... Peak of linoleic acid

0188628

# INTERNATIONAL SEARCH REPORT

International Application No.   PCT/JP85/00408

**I. CLASSIFICATION OF SUBJECT MATTER** (if several classification symbols apply, indicate all) [3]

According to International Patent Classification (IPC) or to both National Classification and IPC

Int. Cl$^4$ C12P 7/64 //C12N 9/20, (C12P 7/64, C12R 1:44)

**II. FIELDS SEARCHED**

| Minimum Documentation Searched [4] | |
|---|---|
| Classification System | Classification Symbols |
| IPC | C12P 7/64, C12N 9/20 |

| Documentation Searched other than Minimum Documentation to the Extent that such Documents are Included in the Fields Searched [5] |
|---|
| |

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| X | JP, A, 56-140887 (Maruho Kabushiki Kaisha), 4 November 1981 (04. 11. 81) (Family: none) | 1 |
| X | JP, A, 55-42532 (Maruho Kabushiki Kaisha), 25 March 1980 (25. 03. 80) (Family: none) | 1 |
| X | Chemical Abstracts, Vol. 81, No.15 14 October 1974 (14. 10. 74) (Columbus, Ohio, U.S.A.) Vadehra, D.V. "Staphylococcus Lipases" Page 112, right column, See Abstruct No. 87058u Lipids 1974, 9(3), 158-165 (Eng.) | 1 |

* Special categories of cited documents: [16]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

**IV. CERTIFICATION**

| Date of the Actual Completion of the International Search [2] | Date of Mailing of this International Search Report [2] |
|---|---|
| October 4, 1985 (04. 10. 85) | October 28, 1985 (28. 10. 85) |
| International Searching Authority [1] | Signature of Authorized Officer [20] |
| Japanese Patent Office | |

Form PCT/ISA/210 (second sheet) (October 1981)